(19) Europäisches Patentamt
European Patent Office
Office européen des brevets

(11) **EP 2 912 110 B1**

(12) **EUROPÄISCHE PATENTSCHRIFT**

(45) Veröffentlichungstag und Bekanntmachung des Hinweises auf die Patenterteilung:
**05.12.2018 Patentblatt 2018/49**

(51) Int Cl.:
*C08K 5/13* *(2006.01)*      *C08K 5/14* *(2006.01)*
*A61L 15/60* *(2006.01)*     *A61L 15/42* *(2006.01)*
*B01J 20/26* *(2006.01)*     *C08F 220/06* *(2006.01)*
*A61L 15/24* *(2006.01)*     *B01J 20/30* *(2006.01)*
*C08K 3/011* *(2018.01)*

(21) Anmeldenummer: **13782712.7**

(22) Anmeldetag: **24.10.2013**

(86) Internationale Anmeldenummer:
**PCT/EP2013/072229**

(87) Internationale Veröffentlichungsnummer:
**WO 2014/064176 (01.05.2014 Gazette 2014/18)**

(54) **GERUCHS- UND FARBSTABILE WASSERABSORBIERENDE ZUSAMMENSETZUNG**

SCENT- AND COLOUR-STABLE WATER-ABSORBING COMPOSITION

COMPOSITION ABSORBANT L'EAU À ODEUR ET COULEUR STABLES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **24.10.2012 DE 102012219378**

(43) Veröffentlichungstag der Anmeldung:
**02.09.2015 Patentblatt 2015/36**

(73) Patentinhaber: **Evonik Degussa GmbH**
**45128 Essen (DE)**

(72) Erfinder:
• **WATTEBLED, Laurent**
**40589 Düsseldorf (DE)**
• **PILLAT, Jessica**
**47798 Krefeld (DE)**
• **MAUS, Lisa**
**47228 Duisburg (DE)**
• **SMITH, Scott**
**40489 Düsseldorf (DE)**
• **HARREN, Jörg**
**52499 Baesweiler (DE)**

(56) Entgegenhaltungen:
EP-A2- 0 942 014          WO-A1-95/05856
WO-A1-99/08726           WO-A1-03/051940
WO-A1-2004/085496        WO-A2-2009/066255
WO-A2-2011/023560        GB-A- 2 377 890
JP-A- H04 331 205         JP-A- 2005 225 921
US-A1- 2005 085 604

• BUCHHOLZ F L ET AL: "MODERN SUPERABSORBENT POLYMER TECHNOLOGY", 1 January 1998 (1998-01-01), MODERN SUPERABSORBENT POLYMER TECHNOLOGY, WILEY-VCH, NEW YORK [U.A.], PAGE(S) I - IV,24,39, XP003035248, ISBN: 978-0-471-19411-8

**Beschreibung**

Die vorliegende Erfindung betrifft eine wasserabsorbierende Zusammensetzung, ein Verfahren zur Herstellung einer wasserabsorbierenden Zusammensetzung sowie die Verwendung einer wasserabsorbierenden Zusammensetzung.

Superabsorber sind wasserunlösliche, vernetzte Polymere, die in der Lage sind, unter Quellung und Ausbildung von Hydrogelen große Mengen an Wasser, wässrigen Flüssigkeiten, insbesondere Körperflüssigkeiten, vorzugsweise Urin oder Blut, aufzunehmen und unter Druck zurückzuhalten. Superabsorber absorbieren vorzugsweise mindestens das 100-fache ihres Eigengewichts an Wasser. Weitere Einzelheiten zu Superabsorbern sind in "Modem Superabsorbent Polymer Technology", F. L. Buchholz, A. T. Graham, Wiley-VCH, 1998" offenbart. Durch diese charakteristischen Eigenschaften sind diese wasserabsorbierenden Polymere hauptsächlich in Sanitärartikeln wie beispielsweise Babywindeln, Inkontinenzprodukten oder Damenbinden eingearbeitet.

Bei den gegenwärtig kommerziell verfügbaren Superabsorbern handelt es sich im wesentlichen um vernetzte Polyacrylsäuren oder vernetzte Stärke-Acrylsäure-Pfropfpolymerisate, bei denen die Carboxylgruppen teilweise mit Natronlauge oder Kalilauge neutralisiert sind. Diese sind dadurch erhältlich, dass monomere Acrylsäure bzw. deren Salze in Gegenwart geeigneter Vernetzungsmittel radikalisch polymerisiert werden. Dabei können unterschiedliche Polymerisationsverfahren zur Anwendung gelangen, wie beispielsweise die Lösungspolymerisation, die Emulsionspolymerisation oder die Suspensionspolymerisation. Letztendlich werden durch diese unterschiedlichen Verfahren wasserabsorbierende Polymere in partikulärer Form mit einem Partikeldurchmesser in einem Bereich von 150 bis 850 $\mu$m erhalten, die dann in die Sanitärartikel eingearbeitet werden.

Derartige Superabsorber neigen jedoch dazu, sich bei längerer Lagerung zu verfärben. Zudem wird die Tendenz, dass sich ihr sauberes, frisches weiß honigbraun verfärbt, bei zunehmenden Lagerzeiten, Temperaturen und Luftfeuchtigkeiten noch beschleunigt. Unter gemäßigten klimatischen Verhältnissen, wie sie in den Vereinigten Staaten von Amerika und in Europa herrschen, ist die Verfärbungsgeschwindigkeit eines Superabsorbers allerdings so langsam, dass der Superabsorber bzw. das superabsorberhaltige Erzeugnis normalerweise schon aufgebraucht ist, ehe man eine Farbänderung mit bloßem Auge erkennen kann. In tropischen und subtropischen Klimazonen, wie in Südamerika und Südostasien, verläuft die Verfärbung des Superabsorbers jedoch so schnell, dass eine Farbänderung noch vor dem Gebrauch des Superabsorbers bzw. des superabsorberhaltigen Erzeugnisses eintritt.

Verantwortlich für die Verfärbung des Superabsorbers sind, neben den bei der radikalischen Polymerisation eingesetzten und im Polymerisat verbleibenden Initiatoren, wie beispielsweise Ascorbinsäure und Natriumperoxodisulfat, sowie neben Inhibitoren, die in der Regel in der eingesetzten Acrylsäure zum Zwecke der Verhinderung einer spontan erfolgenden Polymerisation enthalten sind, wie beispielsweise der Monomethylether des Hydroquinons (HQME), auch bestimmte Zusatzstoffe, mit welchen die Superabsorber nach ihrer Herstellung vermischt werden. Als besonders nachteilig für die Farbstabilität haben sich in diesem Zusammenhang Oxidationsmittel wie etwa Natriumpercarbonat erwiesen, welche gemäß der Lehre der WO-A-2009/066255 in partikulärer Form mit Superabsorbern vermischt werden, um die geruchsbildenden Eigenschaften der Superabsorber zu verbessern. Wenn ein Superabsorber beinhaltender Hygieneartikel Flüssigkeiten, wie beispielsweise Urin, absorbiert, so bilden sich in der Regel schon nach kurzer Zeit unangenehme Gerüche, die von den Komponenten des Urins, insbesondere Ammoniak und anderen alkalischen Stickstoff-enthaltenden Verbindungen herrühren. Zudem kann Bakterienwachstum zu Produkten führen, die Geruch verursachen und/oder zu Hautirritationen führen. Um diesem Problem zu begegnen, schlägt WO-A-2009/066255 vor, Oxidationsmittel wie beispielsweise Natriumpercarbonat zusammen mit Bleichaktivatoren in den Hygieneartikel einzubringen. Vorzugsweise werden dabei die Oxidationsmittel zusammen mit den Superabsorbern in den absorbierenden Kern eines Hygieneartikels eingebracht. Dabei zeigt sich jedoch, dass die Farbstabilität der Superabsorber durch die Anwesenheit der Oxidationsmittel erkennbar leidet. Eine verbesserte Geruchsstabilität geht daher einher mit einer verschlechterten Farbstabilität.

EP 0 942 014 A2, JP 2005 225921 A, WO 2004/085496, WO 03/051940 A1 und US 2005/085604 A1 betreffen die Farbstabilität von wasserabsorbierenden Zusammensetzungen. GB 2 377 890 A und WO 99/08726 A1 betreffen die Geruchsvermeidung bei Hygieneartikeln.

Der vorliegenden Erfindung lag die Aufgabe zugrunde, die sich aus dem Stand der Technik ergebenden Nachteile zu überwinden.

Insbesondere lag der vorliegenden Erfindung die Aufgabe zugrunde, eine wasserabsorbierende Zusammensetzung anzugeben, die sowohl durch eine vorteilhafte Geruchsstabilität als auch durch eine vorteilhafte Farbstabilität gekennzeichnet ist.

Auch lag der vorliegenden Erfindung die Aufgabe zugrunde, ein Verfahren anzugeben, mit dem eine derart vorteilhafte wasserabsorbierende Zusammensetzung hergestellt werden kann.

Die Lösung ist eine wasserabsorbierende Zusammensetzung gemäß Anspruch 1, ihre Verwendung gemäß Anspruch 8, und ein Verfahren zur Herstellung einer wasserabsorbierenden Zusammensetzung gemäß Anspruch 5.

Gemäß einer besonderen Ausgestaltung der erfindungsgemäßen wasserabsorbierenden Zusammensetzung liegt das Gewichtsverhältnis der Komponente iii) zur Komponente ii) in einem Bereich von 1 :20 bis 1:1, besonders bevorzugt in einem Bereich von 1:15 bis 1:2 und am meisten bevorzugt in einem Bereich von 1 : 14 bis 1 : 4.

Völlig überraschend wurde festgestellt, dass sich der negative Einfluss von Oxidationsmitteln wie beispielsweise Natriumpercarbonat auf die Farbstabilität von Superabsorbern reduzieren lässt, in dem die Superabsorber zusätzlich mit Polymerisationsinhibitoren gemäß Anspruch 1 wie beispielsweise Hydrochinonmonomethylether (HQME) in Kontakt gebracht werden. Dies ist insofern überraschend, als Polymerisationsinhibitoren wie HQME, welche üblicherweise der zur Herstellung von Superabsorbern eingesetzten Acrylsäure als Additiv zugesetzt werden, um insbesondere eine spontane Polymerisation der Acrylsäure zu verhindern, im Stand der Technik selbst ein negativer Einfluss auf die Farbstabilität zugeschrieben wird.

Die erfindungsgemäße wasserabsorbierende Zusammensetzung beinhaltet als Komponente i) mindestens ein wasserabsorbierendes Polymer.

Erfindungsgemäß bevorzugte wasserabsorbierende Polymere i) sind Fasern, Schäume oder Partikel, wobei Fasern und Partikel bevorzugt und Partikel besonders bevorzugt sind.

Erfindungsgemäß bevorzugte Polymerfasern sind so dimensioniert, dass sie in oder als Garne für Textilien und auch direkt in Textilien eingearbeitet werden können. Es ist erfindungsgemäß bevorzugt, dass die Polymerfasern eine Länge im Bereich von 1 bis 500 mm, bevorzugt 2 bis 500 mm und besonders bevorzugt 5 bis 100 mm und einen Durchmesser im Bereich von 1 bis 200 Denier, bevorzugt 3 bis 100 Denier und besonders bevorzugt 5 bis 60 Denier besitzen.

Erfindungsgemäß bevorzugte partikelförmige wasserabsorbierende Polymere i) sind so dimensioniert, dass sie eine mittlere Teilchengröße gemäß WSP 220.2 im Bereich von 10 bis 3000 $\mu$m, vorzugsweise 20 bis 2000 $\mu$m und besonders bevorzugt 150 bis 850 $\mu$m aufweisen. Dabei ist es insbesondere bevorzugt, dass mindestens 90 Gew.-% der Partikel des wasserabsorbierenden Polymers i) eine Partikelgröße in einem Bereich von 150 bis 850 $\mu$m aufweisen. Gemäß einer besonderen Ausgestaltung der wasserabsorbierenden Polymere i) beträgt der Anteil der Polymerpartikel mit einer Partikelgröße in einem Bereich von 300 bis 600 $\mu$m mindestens 50 Gew.-%, besonders bevorzugt mindestens 65 Gew.-% und am meisten bevorzugt mindestens 80 Gew.-%, bezogen auf das Gesamtgewicht der wasserabsorbierenden Polymere i).

Das wasserabsorbierende Polymer i) basiert vorzugsweise auf vernetzter und mindestens teilweise neutralisierter Polyacrylsäure. In diesem Zusammenhang ist es insbesondere bevorzugt, dass die wasserabsorbierenden Polymere i) zu mindestens 50 Gew.-%, vorzugsweise zu mindestens 70 Gew.-% und darüber hinaus bevorzugt zu mindestens 90 Gew.-%, jeweils bezogen auf das Gewicht der Polymere, auf polymerisierter Acrylsäure basieren. Es ist erfindungsgemäß weiterhin bevorzugt, dass die wasserabsorbierenden Polymere i) zu mindestens 50 Gew.-%, vorzugsweise zu mindestens 70 Gew.-%, jeweils bezogen auf das Gewicht der Polymermaterialien, auf polymerisierter Acrylsäure basieren, die vorzugsweise zu mindestens 20 Mol-%, besonders bevorzugt zu mindestens 50 Mol-% und darüber hinaus bevorzugt in einem Bereich von 60 bis 85 Mol-% neutralisiert ist.

Die wasserabsorbierenden Polymer i) sind vorzugsweise erhältlich durch ein Verfahren beinhaltend die Verfahrensschritte:

a) radikalische Polymerisation einer wässrigen Monomerlösung beinhaltend ein polymerisierbares, monoethylenisch ungesättigtes, eine Säuregruppe-tragendes Monomer ($\alpha$1) oder ein Salz davon, gegebenenfalls ein mit dem Monomer ($\alpha$1) polymerisierbares, monoethylenisch ungesättigtes Monomer ($\alpha$2), sowie mindestens einen Vernetzer ($\alpha$3) unter Erhalt eines Hydrogels;

b) gegebenenfalls Zerkleinern des Hydrogels;

c) Trocknen des gegebenenfalls zerkleinerten Hydrogels unter Erhalt teilchenförmiger, wasserabsorbierender Polymere;

d) gegebenenfalls Zermahlen und Absieben der so erhaltenen teilchenförmigen wasserabsorbierenden Polymere;

e) Oberflächennachvernetzung, der so erhaltenen teilchenförmigen, wasserabsorbierenden Polymere mit einem Vernetzer, der mindestens zwei funktionelle Gruppen aufweist, die mit den Säuregruppen auf der Oberfläche der Polymermaterialien zu reagieren vermögen.

Im Verfahrensschritt a) wird zunächst eine wässrige Monomerlösung beinhaltend ein polymerisierbares, monoethylenisch ungesättigtes, eine Säuregruppetragendes Monomer ($\alpha$1) oder ein Salz davon, gegebenenfalls ein mit dem Monomer ($\alpha$1) polymerisierbares, monoethylenisch ungesättigtes Monomer ($\alpha$2), sowie mindestens einen Vernetzer ($\alpha$3) unter Erhalt eines Polymergels radikalisch polymerisiert. Die monoethylenisch ungesättigten, säuregruppen-tragenden Monomere ($\alpha$1) können teilweise oder vollständig, bevorzugt teilweise neutralisiert sein. Vorzugsweise sind die monoethylenisch ungesättigten, säuregruppen-tragenden Monomere ($\alpha$1) zu mindestens 25 Mol-%, besonders bevorzugt zu mindestens 50 Mol-% und darüber hinaus bevorzugt zu 50-80 Mol-% neutralisiert. In diesem Zusammenhang wird auf DE 195 29 348 A1 verwiesen. Die Neutralisation kann teilweise oder ganz auch nach der Polymerisation erfolgen. Ferner kann die Neutralisation mit Alkalimetallhydroxiden, Erdalkalimetallhydroxiden, Ammoniak sowie Carbonaten und Bicarbonaten erfolgen. Daneben ist jede weitere Base denkbar, die mit der Säure ein wasserlösliches Salz bildet. Auch eine Mischneutralisation mit verschiedenen Basen ist denkbar. Bevorzugt ist die Neutralisation mit einer Kombination aus Carbonaten und/oder Bicarbonaten (die gleichzeitig als Bläh- oder Treibmittel wirken) und Alkalimetallhydroxiden, besonders bevorzugt mit einer Kombination aus Natriumcarbonat und Natriumhydroxid.

Bevorzugte monoethylenisch ungesättigte, säuregruppen-tragende Monomere (α1) sind vorzugsweise diejenigen Verbindungen, die in der WO 2004/037903 A2, als ethylenisch ungesättigte, säuregruppenhaltige Monomere (α1) genannt werden. Besonders bevorzugte monoethylenisch ungesättigte, säuregruppen-tragende Monomere (α1) sind Acrylsäure und Methacrylsäure, wobei Acrylsäure am meisten bevorzugt ist.

Als mit den Monomeren (α1) copolymerisierbare, monoethylenisch ungesättigte Monomere (α2) können Acrylamide, Methacrylamide oder Vinylamide eingesetzt werden. Weitere bevorzugte Co-Monomere sind insbesondere diejenigen, die in der -tragende Monomere (α1) sind vorzugsweise diejenigen Verbindungen, die in der WO 2004/037903 A2 als Co-Monomere (α2) genannt werden.

Als Vernetzer (α3) werden vorzugsweise ebenfalls diejenigen Verbindungen eingesetzt, die in der WO 2004/037903 A2 als Vernetzer (α3). Unter diesen Vernetzern sind wasserlösliche Vernetzer besonders bevorzugt. Am meisten bevorzugt sind dabei N,N'-Methylenbisacrylamid, Polyethylenglykoldi-(meth)acrylate, Triallylmethylammoniumchlorid, Tetraallylammonium-chlorid sowie mit 9 Mol Ethylenoxid pro Mol Acrylsäure hergestelltes Allylnonaethylenglyko lacrylat.

Neben den Monomeren (α1) und gegebenenfalls (α2) sowie dem mindestens einen Vernetzer (α3) kann die Monomerlösung auch wasserlösliche Polymere (α4) beinhalten. Bevorzugte wasserlösliche Polymere umfassend teil- oder vollverseiften Polyvinylalkohol, Polyvinylpyrrolidon, Stärke oder Stärkederivate, Polyglykole oder Polyacrylsäure. Das Molekulargewicht dieser Polymere ist unkritisch, solange sie wasserlöslich sind. Bevorzugte wasserlösliche Polymere sind Stärke oder Stärkederivate oder Polyvinylalkohol. Die wasserlöslichen Polymere, vorzugsweise synthetische wie Polyvinylalkohol, können nicht nur als Pfropfgrundlage für die zu polymerisierenden Monomere dienen. Denkbar ist auch, diese wasserlöslichen Polymere erst nach der Polymerisation mit dem Hydrogel oder dem bereits getrockneten, wasserabsorbierenden Polymer zu vermischen.

Weiterhin kann die Monomerlösung auch Hilfsmittel (α5) enthalten, wobei zu diesen Hilfsmitteln insbesondere die für die Polymerisation gegebenenfalls erforderlichen Initiatoren oder Komplexbildner, wie beispielsweise EDTA, gehören.

Als Lösungsmittel für die Monomerlösung kommen Wasser, organische Lösungsmittel oder Gemische aus Wasser und organischen Lösungsmitteln in Betracht, wobei die Wahl des Lösungsmittels insbesondere auch von der Art und Weise der Polymerisation abhängt.

Die relative Menge an Monomeren (α1) und (α2) sowie an Vernetzern (α3) und wasserlöslichen Polymeren (α4) und Hilfsmittel (α5) in der Monomerlösung wird vorzugsweise so gewählt, dass das im Verfahrensschritt iii) nach dem Trocknen erhaltene teilchenförmige, wasserabsorbierende Polymer

- zu 20 bis 99,999 Gew.-%, bevorzugt zu 55 bis 98,99 Gew.-% und besonders bevorzugt zu 70 bis 98,79 Gew.-% auf den Monomeren (α1),
- zu 0 bis 80 Gew.-%, vorzugsweise zu 0 bis 44,99 Gew.-% und besonders bevorzugt zu 0,1 bis 44,89 Gew.-% auf den Monomeren (α2),
- zu 0 bis 5 Gew.-%, vorzugsweise zu 0,001 bis 3 Gew.-% und besonders bevorzugt zu 0,01 bis 2,5 Gew.-% auf den Vernetzern (α3),
- zu 0 bis 30 Gew.-%, vorzugsweise zu 0 bis 5 Gew.-% und besonders bevorzugt zu 0,1 bis 5 Gew.-% auf den wasserlöslichen Polymeren (α4),
- zu 0 bis 20 Gew.-%, vorzugsweise zu 0 bis 10 Gew.-% und besonders bevorzugt zu 0,1 bis 8 Gew.-% auf den Hilfsmitteln (α5), und
- zu 0,5 bis 25 Gew.-%, vorzugsweise zu 1 bis 10 Gew.-% und besonders bevorzugt zu 3 bis 7 Gew.-% auf Wasser (α6)

basiert, wobei die Summe der Gewichtsmengen (α1) bis (α6) 100 Gew.-% beträgt. Optimale Werte für die Konzentration insbesondere der Monomere, der Vernetzer und wasserlöslichen Polymere in der Monomerlösung können durch einfache Vorversuche ermittelt oder aber auch dem Stand der Technik, insbesondere den Druckschriften US 4,286,082, DE-A-27 06 135, US 4,076,663, DE-A-35 03 458, DE 40 20 780 C1, DE-A-42 44 548, DE-A-43 33 056 und DE-A-44 18 818 entnommen werden. Zur radikalischen Polymerisation der Monomerlösung können grundsätzlich alle dem Fachmann bekannten Polymerisationsverfahren in Betracht kommen. Beispielsweise sind in diesem Zusammenhang Lösungspolymerisation, die vorzugsweise in Knetreaktoren wie Extrudern oder kontinuierlich auf einem Polymerisationsband erfolgt, Spraypolymerisation, inverse Emulsionspolymerisation und inverse Suspensionspolymerisation zu nennen.

Bevorzugt wird die Lösungspolymerisation in Wasser als Lösungsmittel durchgeführt. Die Lösungspolymerisation kann kontinuierlich oder diskontinuierlich erfolgen. Aus dem Stand der Technik ist ein breites Spektrum von Variationsmöglichkeiten hinsichtlich Reaktionsverhältnisse wie Temperaturen, Art und Menge der Initiatoren als auch der Reaktionslösung zu entnehmen. Typische Verfahren sind in den folgenden Patentschriften beschrieben: US 4,286,082, DE-A-27 06 135 A1, US 4,076,663, DE-A-35 03 458, DE 40 20 780 C1, DE-A-42 44 548, DE-A-43 33 056, DE-A-44 18 818.

Die Polymerisation wird wie allgemein üblich durch einen Initiator ausgelöst. Als Initiatoren zur Initiierung der Polymerisation können alle unter den Polymerisationsbedingungen Radikale bildende Initiatoren verwendet werden, die üblicherweise bei der Herstellung von Superabsorbern eingesetzt werden. Auch eine Initiierung der Polymerisation durch Einwirkung von Elektronenstrahlen auf die polymerisierbare, wässrige Mischung ist möglich. Die Polymerisation kann allerdings auch in Abwesenheit von Initiatoren der oben genannten Art durch Einwirkung energiereicher Strahlung in Gegenwart von Photoinitiatoren ausgelöst werden. Polymerisationsinitiatoren können in der Monomerlösung gelöst oder dispergiert enthalten sein. Als Initiatoren kommen sämtliche dem Fachmann bekannte in Radikale zerfallende Verbindungen in Betracht. Hierunter fallen insbesondere diejenigen Initiatoren, die bereits in der WO-A-2004/037903 als mögliche Initiatoren genannt werden. Besonders bevorzugt wird zur Herstellung der wasserabsorbierenden Polymergebilde ein Redoxsystem bestehend aus Wasserstoffperoxid, Natriumperoxodisulfat und Ascorbinsäure eingesetzt.

Auch die inverse Suspensions- und Emulsionspolymerisation kann zur Herstellung der wasserabsorbierenden Polymere i) angewendet werden. Gemäß diesen Prozessen wird eine wässrige, teilneutralisierte Lösung der Monomeren ($\alpha$1) sowie gegebenenfalls beinhaltend die weiteren Monomere ($\alpha$2), die wasserlöslichen Polymere ($\alpha$4) und Hilfsmittel ($\alpha$5), mit Hilfe von Schutzkolloiden und/oder Emulgatoren in einem hydrophoben, organischen Lösungsmittel dispergiert und durch Radikalinitiatoren die Polymerisation gestartet. Die Vernetzer ($\alpha$3) sind entweder in der Monomerlösung gelöst und werden mit dieser zusammen dosiert oder aber separat und gegebenenfalls während der Polymerisation zugefügt. Gegebenenfalls erfolgt die Zugabe eines wasserlöslichen Polymeren ($\alpha$4) als Pfropfgrundlage über die Monomerlösung oder durch direkte Vorlage in die Ölphase. Anschließend wird das Wasser azeotrop aus dem Gemisch entfernt und das Polymerisat abfiltriert. Weiterhin kann sowohl bei der Lösungspolymerisation als auch bei der inversen Suspensions- und Emulsionspolymerisation die Vernetzung durch Einpolymerisation des in der Monomerlösung gelösten polyfunktionellen Vernetzers ($\alpha$3) und/oder durch Reaktion geeigneter Vernetzer mit funktionellen Gruppen des Polymeren während der Polymerisationsschritte erfolgen. Die Verfahren sind beispielsweise in den Veröffentlichungen US 4,340,706, DE-A-37 13 601, DE-A-28 40 010 und WO-A-96/05234 beschrieben.

Im Verfahrensschritt b) wird das im Verfahrensschritt a) erhaltene Hydrogel gegebenenfalls zerkleinert, wobei dieses Zerkleinern insbesondere dann erfolgt, wenn die Polymerisation mittels einer Lösungspolymerisation durchgeführt wird. Das Zerkleinern kann durch dem Fachmann bekannte Zerkleinerungsvorrichtungen, wie etwa einem Fleischwolf, erfolgen.

Im Verfahrensschritt c) wird das gegebenenfalls zuvor zerkleinerte Hydrogel getrocknet. Die Trocknung des Hydrogels erfolgt vorzugsweise in geeigneten Trocknern oder Öfen. Beispielhaft seien Drehrohröfen, Wirbelbetttrockner, Tellertrockner, Paddeltrockner oder Infrarottrockner genannt. Weiterhin ist es erfindungsgemäß bevorzugt, dass die Trocknung des Hydrogels im Verfahrensschritt c) bis zu einem Wassergehalt von 0,5 bis 25 Gew.-%, vorzugsweise von 1 bis 10 Gew.-% erfolgt, wobei die Trocknungstemperaturen üblicherweise in einem Bereich von 100 bis 200°C liegen.

Im Verfahrensschritt d) können die im Verfahrensschritt c) erhaltenen, teilchenförmigen, wasserabsorbierenden Polymere insbesondere dann, wenn sie durch Lösungspolymerisation erhalten wurden, noch zermahlen und auf die eingangs genannte Wunschkorngröße abgesiebt werden. Das Zermahlen der getrockneten, wasserabsorbierenden Polymere erfolgt vorzugsweise in geeigneten, mechanischen Zerkleinerungsvorrichten, wie etwa einer Kugelmühle, während das Absieben beispielsweise durch Verwendung von Sieben mit geeigneter Maschenweite erfolgen kann.

Im Verfahrensschritt e) werden die gegebenenfalls zermahlenen und abgesiebten teilchenförmigen, wasserabsorbierenden Polymere mit einem Vernetzer, der mindestens zwei funktionelle Gruppen aufweist, die mit den Säuregruppen auf der Oberfläche der Polymermaterialien zu reagieren vermögen, oberflächennachvernetzt, wobei gegebenenfalls die Oberfläche der Polymere vor, während oder nach der Nachvernetzung mit Aluminiumsalzen, vorzugsweise mit Aluminiumlactat und/oder Aluminiumsulfat, in Kontakt gebracht werden kann.

Zur Oberflächennachvernetzung werden die getrockneten und gegebenenfalls zermahlenen und abgesiebten teilchenförmigen, wasserabsorbierenden Polymere aus den Verfahrensschritten c) oder d) oder aber das noch nicht getrocknete, jedoch vorzugsweise bereits zerkleinerte Hydrogel aus dem Verfahrensschritt b) mit einem vorzugsweise organischen, chemischen Oberflächennachvernetzer in Kontakt gebracht. Dabei wird der Nachvernetzer insbesondere dann, wenn er unter den Nachvernetzungsbedingungen nicht flüssig ist, vorzugsweise in Form eines Fluids umfassend den Nachvernetzer sowie ein Lösungsmittel mit dem teilchenförmigen wasserabsorbierenden Polymer bzw. dem gegebenenfalls zerkleinerten Hydrogel in Kontakt gebracht. Als Lösungsmittel werden dabei vorzugsweise Wasser, mit Wasser mischbare organische Lösungsmittel wie etwa Methanol, Ethanol, 1-Propanol, 2- Propanol oder 1-Butanol oder Mischungen aus mindestens zwei dieser Lösungsmittel eingesetzt, wobei Wasser als Lösungsmittel am meisten bevorzugt ist. Weiterhin ist es bevorzugt, dass der Nachvernetzer in dem Fluid in einer Menge in einem Bereich von 5 bis 75 Gew.-%, besonders bevorzugt 10 bis 50 Gew.-% und am meisten bevorzugt 15 bis 40 Gew.-%, bezogen auf das Gesamtgewicht des Fluids, enthalten ist.

Das in Kontakt bringen des teilchenförmigen, wasserabsorbierenden Polymers bzw. des gegebenenfalls zerkleinerten Hydrogels mit dem Fluid beinhaltend den Nachvernetzer erfolgt vorzugsweise durch gutes Vermischen des Fluids mit dem Polymermaterial bzw. dem Hydrogel.

Geeignete Mischaggregate zum Aufbringen des Fluids sind z. B. der Patterson Kelley-Mischer, DRAIS-Turbulenzmischer, Lödigemischer, Ruberg-Mischer, Schneckenmischer, Tellermischer und Wirbelschichtmischer sowie kontinuierlich arbeitende senkrechte Mischer, in denen das Polymermaterial mittels rotierender Messer in schneller Frequenz gemischt wird (Schugi-Mischer).

Das teilchenförmige, wasserabsorbierende Polymer bzw. das gegebenenfalls zerkleinerte Hydrogel wird bei der Nachvernetzung vorzugsweise mit höchstens 20 Gew. -%, besonders bevorzugt mit höchstens 15 Gew.-%, darüber hinaus bevorzugt mit höchstens 10 Gew. -%, darüber hinaus noch mehr bevorzugt mit höchstens 5 Gew.-% an Lösungsmittel, vorzugsweise Wasser, in Kontakt gebracht.

Bei Polymermaterialien in der Form von vorzugsweise kugelförmigen Partikeln ist es erfindungsgemäß weiterhin bevorzugt, dass das in Kontakt bringen derart erfolgt, dass lediglich der Aussenbereich, nicht jedoch der innere Bereich der teilchenförmigen Polymere mit dem Fluid und somit dem Nachvernetzer in Kontakt gebracht werden.

Als Nachvernetzer werden vorzugsweise Verbindungen verstanden, die mindestens zwei funktionelle Gruppen aufweisen, die mit den Säuregruppen auf der Oberfläche des Polymers in einer Kondensationsreaktion (=Kondensationsvernetzer), in einer Additionsreaktion oder in einer Ringöffnungsreaktion reagieren können. Als Nachvernetzer sind diejenigen bevorzugt, die in WO-A-2004/037903 als Vernetzer der Vernetzerklassen II genannt wurden.

Unter diesen Verbindungen sind als Nachvernetzer besonders bevorzugt Kondensationsvernetzer wie beispielsweise Diethylenglykol, Triethylenglykol, Polyethylenglykol, Glyzerin, Polyglyzerin, Propylenglykol, Diethanolamin, Triethanolamin, Polyoxypropylen, Oxyethylen-Oxypropylen-Blockcopolymere, Sorbitanfettsäureester, Polyoxyethylensorbitanfettsäureester, Trimethylol-propan, Pentaerytrit, Polyvinylalkohol, Sorbitol, 1,3-Dioxolan-2-on (Ethylencarbonat), 4-Methyl-1,3-dioxolan-2-on (Propylencarbonat), 4,5-Dimethyl-1,3-dioxolan-2-on, 4,4-Dimethyl-1,3-dioxo-lan-2-on, 4-Ethyl-1,3-dioxolan-2-on, 4-Hydroxymethyl-1,3-dioxolan-2-on, 1,3-Dioxan-2-on, 4-Methyl-1,3-dioxan-2-on, 4,6-Dimethyl-1,3-dioxan-2-on sowie 1,3-Dioxolan-2-on.

Nachdem das teilchenförmige, wasserabsorbierende Polymere bzw. die gegebenenfalls zerkleinerten Hydrogele mit dem Nachvernetzer bzw. mit dem Fluid beinhaltend den Nachvernetzer in Kontakt gebracht wurden, werden sie auf eine Temperatur im Bereich von 50 bis 300°C, vorzugsweise 75 bis 275°C und besonders bevorzugt 150 bis 250°C erhitzt, so dass, vorzugsweise wodurch, der Außenbereich der Teilchen des Polymers im Vergleich zum Innenbereich stärker vernetzt (=Nachvernetzung) und, sofern ein Hydrogel eingesetzt werden, diese zugleich auch getrocknet werden. Die Zeitdauer der Wärmebehandlung wird durch die Gefahr, dass das gewünschte Eigenschaftsprofil des Polymers infolge von Hitzeeinwirkung zerstört wird, begrenzt.

Vor, während oder nach der Nachvernetzung kann zudem die Oberfläche der Polymere bzw. des gegebenenfalls zerkleinerten Hydrogels mit Aluminiumsalzen, vorzugsweise mit Aluminiumlactat, in Kontakt gebracht. Besonders bevorzugt ist es, dass die Behandlung mit den Aluminiumsalzen zeitgleich mit der Oberflächennachvernetzung durchgeführt wird, indem eine vorzugsweise wässrige Lösung beinhaltend den Nachvernetzter sowie das bzw. die Aluminiumsalze, vorzugsweise Aluminiumlactat, mit dem wasserabsorbierenden Polymer bzw. mit dem Hydrogel in Kontakt gebracht und dann erhitzt wird.

Dabei ist es bevorzugt, dass die Aluminiumsalze in einer Menge in einem Bereich von 0,01 bis 30 Gew.-%, besonders bevorzugt in einer Menge in einem Bereich von 0,1 bis 20 Gew.-% und darüber hinaus bevorzugt in einer Menge in einem Bereich von 0,3 bis 5 Gew.-% (sofern als Hydrat vorliegend, wasserfrei berechnet), jeweils bezogen auf das Gewicht des wasserabsorbierenden Polymers bzw. des gegebenenfalls zerkleinerten Hydrogels, mit dem Polymer bzw. dem Hydrogel in Kontakt gebracht wird.

Bevorzugte Aluminiumsalze sind insbesondere $AlCl_3 \times 6H_2O$, $NaAl(SO_4)_2 \times 12\ H_2O$, $KAl(SO_4)_2 \times 12\ H_2O$ oder $Al_2(SO_4)_3 \times$ 14-18 $H_2O$, Aluminiumlactat oder aber wasserunlösliche Aluminiumverbindungen, wie etwa Aluminiumoxide, beispielsweise $Al_2O_3$, oder Aluminate. Besonders bevorzugt werden Aluminiumlactat, Aluminiumsulfat oder Michungen aus Aluminiumlactat und Aluminiumsulfat eingesetzt.

Weiterhin beinhaltet die erfindungsgemäße Zusammensetzung als Komponente ii) mindestens ein Oxidationsmittel, wobei das Oxidationsmittel ein Percarbonat ist.

Erfindungsgemäß besonders bevorzugt ist der Einsatz von Natriumpercarbonat als Oxidationsmittel.

Weiterhin ist es erfindungsgemäß bevorzugt, dass das Oxidationsmittel ebenfalls in partikulärer Form in der erfindungsgemäßen Zusammensetzung enthalten ist, wobei es sich als vorteilhaft erwiesen hat, wenn mindestens 80 Gew.-%, noch mehr bevorzugt mindestens 90 Gew.-% und am meisten bevorzugt mindestens 100 Gew.-% der Partikel des Oxidationsmittels, vorzugsweise der Natriumpercarbonat-Partikel, eine Partikelgröße in einem Bereich von 400 bis 800 $\mu$m aufweisen, wobei das durch Siebanalyse bestimmte Gewichtsmittel der Partikelgröße vorzugsweise in einem Bereich von 500 bis 800 $\mu$m liegt. Weiterhin beinhaltet die erfindungsgemäße wasserabsorbierende Zusammensetzung als Komponente iii) mindestens einen Inhibitor, der radikalische Polymerisationen hemmt, insbesondere einen Inhibitor, der die radikalische Polymerisation von Acrylsäure hemmt, wobei es sich bei diesem Inhibitor erfindungsgemäß um Hydrochinon oder ein Hydrochinonderivat handelt.

Bevorzugte Derivate des Hydrochinons sind in diesem Zusammenhang die Mono- oder Diether-Derivate.

Erfindungsgemäß Inhibitoren sind ausgewählt aus der Gruppe bestehend aus Hydrochinon, Hydrochinonmonomethylether (HQME), 1,4-Dimethoxybenzol, 4.4'-Oxidiphenol und einer Mischung aus mindestens zwei davon, wobei HQME als Inhibitor am meisten bevorzugt ist.

Die erfindungsgemäße wasserabsorbierende Zusammensetzung zeichnet sich vorzugsweise dadurch aus, dass der Inhibitor iii) nicht gleichmäßig innerhalb des vorzugsweise partikulären wasserabsorbierenden Polymers verteilt ist (eine solche gleichmäßige Verteilung wird erhalten, wenn die Zusammensetzung ausschließlich einen Inhibitor beinhaltet, der bereits vor der Polymerisation in der Acrylsäure als Additiv enthalten war), sondern im Oberflächenbereich in höherer Konzentration vorliegt als im Kern des vorzugsweise partikulären wasserabsorbierenden Polymers (eine solche ungleichmäßige Verteilung wird erhalten, wenn die Zusammensetzung auch einen Inhibitor beinhaltet, der erst nach Abschluss der Polymerisationsreaktion, insbesondere erst nach dem Verfahrensschritt c) und insbesondere erst nach dem Verfahrensschritt e) des vorstehen beschrieben Verfahrens zur Herstellung der wasserabsorbierenden Polymere mit dem partikulären wasserabsorbierenden Polymer vorzugsweise durch einfaches Vermischen in Kontakt gebracht wird).

Gemäß einer besonderen Ausführungsform der erfindungsgemäßen wasserabsorbierenden Zusammensetzung kann diese neben den wasserabsorbierenden Polymeren i), dem Oxidationsmittel ii) und dem Inhibitor iii) als weitere Komponente iv) mindestens einen Bleichaktivator beinhalten, wobei als Bleichaktivator insbesondere diejenigen Bleichaktivatoren in Betracht kommen, die in der WO-A-2009/066255 als geeignete Bleichaktivatoren ("*bleach activator*") genannt werden. Bevorzugte Bleichaktivatoren sind in diesem Zusammenhang ausgewählt aus der Gruppe bestehend aus Tetraacetylethylendiamin (TAED), Benzoylcaprolactam (BzCL), 4-Nitrobenzoylcaprolactam, S-Chlorobenzoylcaprolactam, Benzoyloxybenzolsulphonat (BOBS), Nonanoyloxybenzolsulphonat (NOBS), Phenylbenzoat (PhBz), Decanoyloxybenzolsulphonat ($C_{10}$-OBS), Benzoylvalerolactam (BZVL), Octanoyloxybenzolsulphonat ($C_8$-OBS) und Mischungen aus mindestens zwei davon, wobei der Einsatz von Tetraacetylendimain (TAED), Nonanoylbenzolsulphonat (NOBS) oder einer Mischung aus mindestens zwei davon besonders bevorzugt ist. Der Bleichaktivator kann dabei, wie dies ebenfalls in der WO-A-2009/066255 beschrieben ist, gemäß der Lehre der WO-A-2005/080542 über geeignete Bindemittel an einen partikulären Träger gebunden sein. Die Menge an Bleichaktivatoren, sofern dieses in der erfindungsgemäßen wasserabsorbierenden Zusammensetzung enthalten sind, ist vorzugsweise so bemessen, dass das Molverhältnis von Wasserstoffperoxid (welches von der Wasserstoffperoxid-Quelle bereitgestellt wird) zum Bleichaktivator in einem Bereich von 100: 1 bis 1 : 1, besonders bevorzugt in einem Bereich von 80 : 1 bis 5 : 1 liegt.

Vorzugsweise ist die erfindungsgemäße wasserabsorbierende Zusammensetzung durch einen gemäß der hierin beschriebenen Testmethode bestimmten ΔL-Wert von höchstens 10, besonders bevorzugt von höchstens 7,5 und am meisten bevorzugt von höchstens 5 nach einer Lagerung der wasserabsorbierenden Zusammensetzung für 10 Tage bei 70°C und einer relativen Luftfeuchtigkeit von 75 % gekennzeichnet. Weiterhin ist es erfindungsgemäß bevorzugt, dass die erfindungsgemäße wasserabsorbierende Zusammensetzungen einen ΔL-Wert aufweist, der um mindestens 10, vorzugsweise mindestens 20 und am meisten bevorzugt mindestens 30 kleiner ist als der ΔL-Wert der entsprechenden Zusammensetzung ohne die Komponente iii), nach einer Lagerung der wasserabsorbierenden Zusammensetzung für 10 Tage bei 70°C und einer relativen Luftfeuchtigkeit von 75 % gekennzeichnet.

Die Erfindung betrifft auch ein Verfahren gemäß Anspruch 5.

Im Verfahrensschritt (I) wird zunächst mindestens ein wasserabsorbierendes Polymer bereitgestellt, wobei als wasserabsorbierendes Polymer diejenigen wasserabsorbierenden Polymere bevorzugt sind, die bereits eingangs im Zusammenhang mit der erfindungsgemäßen wasserabsorbierenden Zusammensetzung als bevorzugte wasserabsorbierende Polymere beschrieben wurden. Vorzugsweise erfolgt die Bereitstellung der wasserabsorbierenden Polymere mittels des eingangs beschriebenen Verfahrens umfassend die Verfahrensschritte a) bis e). In diesem Zusammenhang ist es insbesondere bevorzugt, dass zumindest der Verfahrensschritt (III), vorzugsweise jedoch die Verfahrensschritte (II) und (III), erst nach Abschluss der Nachvernetzungsreaktion gemäß Verfahrensschritt e) des eingangs beschriebenen Verfahrens zur Herstellung der wasserabsorbierenden Polymere durchgeführt wird bzw. werden. In den Verfahrensschritten II) bzw. III) wird dann das wasserabsorbierende Polymer, vorzugsweise die Partikel des wasserabsorbierenden Polymers, mit dem Oxidationsmittel bzw. mit dem Inhibitor in Kontakt gebracht, wobei als Oxidationsmittel und als Inhibitor wiederum diejenigen Verbindungen bevorzugt sind, die bereits eingangs im Zusammenhang mit der erfindungsgemäßen wasserabsorbierenden Zusammensetzung als bevorzugtes Oxidationsmittel bzw. als bevorzugter Inhibitor genannt wurden.

Sowohl das Oxidationsmittel als auch der Inhibitor können dabei als Feststoff oder in Form einer Lösung oder Dispersion mit dem wasserabsorbierenden Polymer in Kontakt gebracht werden. Weiterhin können das Oxidationsmittel und der Inhibitor in einer gemeinsamen Feststoffmischung oder aber in einer gemeinsamen Lösung oder Dispersion oder aber getrennt voneinander nacheinander oder zeitgleich mit dem wasserabsorbierenden Polymer in Kontakt gebracht werden, wobei zum Zwecke des in Kontakt bringens übliche, dem Fachmann für diesen Zweck geeignet erscheinende Mischvorrichtungen eingesetzt werden können.

Die relativen Mengen, in denen das wasserabsorbierende Polymer, das Oxidationsmittel und der Inhibitor eingesetzt werden, ist vorzugsweise so bemessen, dass eine wasserabsorbierende Zusammensetzung beinhaltend

i) 91,2 bis 98,9 Gew.-% und am meisten bevorzugt 94,5 bis 97,8 Gew.-% des mindestens einen wasserabsorbierenden Polymers;

ii) 1 bis 8 Gew.-% und am meisten bevorzugt 2 bis 5 Gew.-% des mindestens einen Oxidationsmittels;

iii) 0,1 bis 0,75 Gew.-% und am meisten bevorzugt 0,2 bis 0,5 Gew.-% des mindestens einen Inhibitor, der radikalische Polymerisationen hemmt,

erhalten wird, wobei die Gewichtsmengen jeweils auf das Gesamtgewicht der wasserabsorbierenden Zusammensetzung bezogen sind.

Gemäß einer besonderen Ausgestaltung des erfindungsgemäßen Verfahrens beinhaltet dieses als weiteren Verfahrensschritt:

IV) das in Kontakt bringen des mindestens einen wasserabsorbierenden Polymers mit mindestens einem Bleichaktivator,

wobei als Bleichaktivator wieder diejenigen Verbindungen bevorzugt sind, die bereits eingangs im Zusammenhang mit der erfindungsgemäßen wasserabsorbierenden Zusammensetzung als bevorzugte Bleichaktivatoren genannt wurden. Auch hinsichtlich der Art und Weise des Zusatzes dieser Bleichaktivatoren sowie der relative Menge, in der diese zum Wasserstoffperoxid eingesetzt werden, wird auf die Ausführungen im Zusammenhang mit der erfindungsgemäßen wasserabsorbierenden Zusammensetzung verwiesen.

Die Beschreibung offenbart auch eine wässrige Zusammensetzung, welche durch das erfindungsgemäße Verfahren erhältlich ist. Vorzugsweise ist die durch das erfindungsgemäße Verfahren erhältliche Zusammensetzung durch einen gemäß der hierin beschriebenen Testmethode bestimmten $\Delta L$-Wert von höchstens 10, besonders bevorzugt von höchstens 7,5 und am meisten bevorzugt von höchstens 5 nach einer Lagerung der wasserabsorbierenden Zusammensetzung für 10 Tage bei 70°C und einer relativen Luftfeuchtigkeit von 75 % gekennzeichnet.

Weiterhin ist es bevorzugt, dass die durch das erfindungsgemäße Verfahren erhältliche wasserabsorbierende Zusammensetzung einen $\Delta L$-Wert aufweist, der um mindestens 10, vorzugsweise mindestens 20 und am meisten bevorzugt mindestens 30 kleiner ist als der $\Delta L$-Wert einer Zusammensetzung, welche durch das entsprechende Verfahren ohne den Verfahrensschritt III) erhalten wurde, nach einer Lagerung der wasserabsorbierenden Zusammensetzung für 10 Tage bei 70°C und einer relativen Luftfeuchtigkeit von 75 % gekennzeichnet. Die Beschreibung offenbart auch einen Verbund, beinhaltend die erfindungsgemäße wasserabsorbierende Zusammensetzung bzw. die durch das erfindungsgemäße Verfahren erhältliche wasserabsorbierende Zusammensetzung und ein Substrat. Es ist dabei bevorzugt, dass die wasserabsorbierende Zusammensetzung bzw. die durch das erfindungsgemäße Verfahren erhältliche wasserabsorbierende Zusammensetzung und das Substrat miteinander fest verbunden sind. Als Substrate sind Folien aus Polymeren, wie beispielsweise aus Polyethylen, Polypropylen oder Polyamid, Metalle, Vliese, Fluff, Tissues, Gewebe, natürliche oder synthetische Fasern, oder andere Schäume bevorzugt. Weiterhin ist es bevorzugt, dass der Verbund mindestens einen Bereich umfasst, welcher die erfindungsgemäße wasserabsorbierende Zusammensetzung bzw. die durch das erfindungsgemäße Verfahren erhältliche wasserabsorbierende Zusammensetzung in einer Menge im Bereich von etwa 15 bis 100 Gew.-%, vorzugsweise etwa 30 bis 100 Gew.-%, besonders bevorzugt von etwa 50 bis 99,99 Gew.-%, ferner bevorzugt von etwa 60 bis 99,99 Gew.-% und darüber hinaus bevorzugt von etwa 70 bis 99 Gew.-%, jeweils bezogen auf das Gesamtgewicht des betreffenden Bereichs des Verbundes, beinhaltet, wobei dieser Bereich vorzugsweise eine Größe von mindestens 0,01 cm$^3$, vorzugsweise mindestens 0,1 cm$^3$ und am meisten bevorzugt mindestens 0,5 cm$^3$ aufweist.

In einer besonders bevorzugten Ausführungsform des Verbundes handelt es sich um einen flächenförmigen Verbund, wie er in der WO 02/056812 A1 als "*absorbent material*" beschrieben ist.

Die Beschreibung offenbart auch ein Verfahren zur Herstellung eines Verbundes, wobei die erfindungsgemäße wasserabsorbierende Zusammensetzung und ein Substrat und gegebenenfalls ein Zusatzstoff miteinander in Kontakt gebracht werden. Als Substrate werden vorzugsweise diejenigen Substrate eingesetzt, die bereits vorstehend im Zusammenhang mit dem Verbund genannt wurden.

Die Beschreibung offenbart auch auch ein Verbund erhältlich nach dem vorstehend beschriebenen Verfahren, wobei dieser Verbund vorzugsweise die gleichen Eigenschaften aufweist wie der vorstehend beschriebene Verbund.

Die Beschreibung offenbart auch chemische Produkte beinhaltend die erfindungsgemäße wasserabsorbierende Zusammensetzung, die durch das erfindungsgemäße Verfahren erhältliche wasserabsorbierende Zusammensetzung oder einen Verbund.

Bevorzugte chemische Produkte sind insbesondere Schäume, Formkörper, Fasern, Folien, Filme, Kabel, Dichtungsmaterialien, flüssigkeitsaufnehmenden Hygieneartikel, insbesondere Windeln und Damenbinden, Träger für pflanzen- oder pilzwachstumsregulierende Mittel oder Pflanzenschutzwirkstoffe, Zusätze für Baustoffe, Verpackungsmaterialien oder Bodenzusätze.

Die Erfindung betrifft auch die Verwendung der erfindungsgemäßen wasserabsorbierenden Zusammensetzung gemäß Anspruch 8. Bei der Verwendung als Träger für pflanzen- oder pilzwachstumsregulierende Mittel oder Pflanzenschutzwirkstoffe ist es bevorzugt, dass die pflanzen- oder pilzwachstumsregulierende Mittel oder Pflanzenschutzwirkstoffe über einen durch den Träger kontrollierten Zeitraum abgegeben werden können.

Die Erfindung wird nun anhand von Testmethoden und nicht limitierenden Beispielen näher erläutert.

TESTMETHODEN

Bestimmung des ΔL-Wertes

Der "L*"-Wert gibt den Grauwert wieder (L = 0 entspricht schwarz, L = 100 entspricht weiß). Diese Skalierung basiert auf den Grundsätzen, welche in ASTM E 308 "*Standart Practice for Computing the Colors of Objects Using the CIE-System*" zusammengefasst sind.

Die Bestimmung des L-Farbwertes erfolgt mittels eines Spektralkolorimeters "*Hunter LabScan XE*" (Hunter Associates Laboratory, Reston, VA, USA) bei folgenden Einstellungen:

| | |
|---|---|
| "Mode" | 0/45 |
| "Area View" | 44,5 mm |
| "Port Size" | 50,8 mm |
| "UV-Filter" | Nominal |

Vor jeder Messung wird der LabScan XE kalibriert, in dem zunächst die zum Gerätezubehör gehörende schwarze Glasplatte zwischen Probenteller und Messöffnung eingeklemmt wird, wobei die Glasplatte auf eine Petrischale (Innendurchmesser 88 mm, Tiefe: 14 mm) gelegt und durch Betätigung des "OK"-Schalters die Kalibrierung mit der schwarzen Glasplatte vollzogen wird. Anschließend wird auf die gleiche Art und Weise die weiße Standardplatte auf die Petrischale gelegt und erneut durch Betätigung des "OK"-Schalters die Kalibierung vollzogen.

Nachdem die Kalibierung durchgeführt wurde, wird der "Read Std"-Schalter gedrückt, um die Funktionsfähigkeit des Messgerätes zu prüfen, wobei dabei die Standardplatte noch nicht entfernt wird. Anschließend wird zur Messung des L-Farbwertes für die Standardplatte der "Read"-Schalter betätigt.

Anschließend wird die Standardplatte entfernt und die Petrischale mit zum zu vermessenden wasserabsorbierenden Polymergebilde gefüllt, wobei die Produktoberfläche mit einem Abstreifer glattgezogen wird. Durch Drücken des "Read Sam"-Schalters wird die Probe vermessen (= $L_0$-Wert). Es werden insgesamt 8 Messungen an unterschiedlichen Stellen der Probe durchgeführt und der Mittelwert gebildet. Anschließend wird die Probe für 10 Tage bei 70°C und einer relativen Luftfeuchtigkeit von 75 % im Klimaschrank gelagert. Im Anschluss an diese Lagerung wird erneut der L-Wert bestimmt (= $L_1$-Wert). Es werden insgesamt 8 Messungen an unterschiedlichen Stellen der Probe durchgeführt und der Mittelwert gebildet.

Es gilt:

$$\Delta L = |L_1 - L_0|$$

Des Weiteren wird auch der ΔL' Wert bestimmt, bei dem es sich um die Differenz aus dem Vergleichbeispiel 1 mit einer Perverbindung ohne Inhibitor und den Beispielen 1 bis 3 mit der Kombination aus einer Perverbindung mit einem Inhibitor handelt.

$$\Delta L` = |\Delta L - \Delta L_i|$$

BEISPIELE

Herstellungsbeispiel 1:

Eine Monomerlösung bestehend aus 320 g Acrylsäure, 248,649 g NaOH (50%ig), 407,022 g entionisiertes Wasser, 0,631 g Polyethylenglykol-300-diacrylat (mit einem Gehalt an wirksamer Substanz von 76,1 Gew.-%) und 1,31 g Polyethylenglykol-500-O-monoallyletheracrylat (mit einem Gehalt an wirksamer Substanz von 73,1 Gew.-%) wurde durch Spülen mit Stickstoff vom gelösten Sauerstoff befreit und auf die Starttemperatur von 4°C abgekühlt. Nach Erreichen der Starttemperatur wurde die Initiatorlösung (0,3 g Natriumperoxodisulfat in 10,0 g $H_2O$, 0,07 g 35%-ige Wasserstoffperoxid-Lösung in 10,0 g $H_2O$ und 0,015 g Ascorbinsäure in 2,0 g $H_2O$) zugesetzt. Nachdem die Endtemperatur von etwa 110°C erreicht war, wurde 20 min nachreagiert und das entstandene Gel wurde mit einem Fleischwolf zerkleinert und bei 150°C 2 Stunden lang im Trockenschrank getrocknet. Das getrocknete Polymerisat wurde grob zerstoßen, mittels einer Schneidmühle SM 100 mit einem 1,5 mm-Sieb gemahlen und auf ein Pulver mit einer Partikelgröße von 150 bis 710 μm gesiebt (=Pulver A).

Das Pulver A wurde mit einer wässrigen Lösung bestehend aus Ethylencarbonat (1 Gew.-% bezogen auf das Pulver A), Aluminiumlactat (0,4 Gew.-% bezogen auf das Pulver A), Aluminiumsulfat (0,3 Gew.-% bezogen auf das Pulver A) und Wasser (3 Gew.-% bezogen auf das Pulver A) in einem Labormischer vermischt und anschließend im Ofen für 90 min. bei 170°C erhitzt (Pulver B).

Vergleichsbeispiel 1 (nur Oxidationsmittel, kein Inhibitor):

50 g des wasserabsorbierenden Polymers aus Herstellungsbeispiel 1 wurden mit 1,5 g Natriumpercarbonat (Q35 Standard HPC der Evonik Industries) für 1 Stunde auf einer Rollbank homogen vermischt.

Beispiel 1 (erfindungsgemäß):

50 g des wasserabsorbierenden Polymers aus Herstellungsbeispiel 1 wurden mit 1,5 g Natriumpercarbonat (Q35 Standard HPC der Evonik Industries) und 0,125 g HQME für 1 Stunde auf einer Rollbank homogen vermischt.

Von den vorstehenden Zusammensetzungen wurden die ΔL-Werte bestimmt. Die Ergebnisse sind in der nachfolgenden Tabelle zusammengefasst:

| Zusammensetzung | ΔL-Wert | ΔL'-Wert |
|---|---|---|
| Herstellungsbeispiel | 13,0 | |
| Vergleichsbeispiel 1 | 34,0 | |
| Beispiel 1 | 1,0 | 33 |

Die Ergebnisse zeigen, dass HQME die durch Natriumpercarbonat hervorgerufene starke Verfärbung (siehe Vergleichsbeispiel 1) deutlich zu reduzieren vermag.

Beispiel 2 (erfindungsgemäß):

50 g des wasserabsorbierenden Polymers aus Herstellungsbeispiel 1 wurden mit 1,5 g Natriumpercarbonat (Q35 Standard HPC der Evonik Industries) und 0,11 g Hydrochinon für 1 Stunde auf einer Rollbank homogen vermischt.

Beispiel 3 (erfindungsgemäß):

50 g des wasserabsorbierenden Polymers aus Herstellungsbeispiel 1 wurden mit 1,5 g Natriumpercarbonat (Q35 Standard HPC der Evonik Industries) und 0,203 g 4,4'-Oxidiphenol für 1 Stunde auf einer Rollbank homogen vermischt.

Von den vorstehenden Zusammensetzungen wurden die ΔL-Werte bestimmt. Die Ergebnisse sind in der nachfolgenden Tabelle zusammengefasst:

| Zusammensetzung | ΔL-Wert | ΔL'-Wert |
|---|---|---|
| Vergleichsbeispiel 1 | 34 | |
| Beispiel 2 | 10 | 24 |
| Beispiel 3 | 5 | 29 |

Die Ergebnisse zeigen, dass neben HQME auch Hydrochinon und 4,4'-Oxidiphenol die durch Natriumpercarbonat hervorgerufene starke Verfärbung (siehe Vergleichsbeispiel 1) deutlich zu reduzieren vermögen.

Vergleichsbeispiel 2 (nur Oxidationsmittel, kein Inhibitor):

50 g des wasserabsorbierenden Polymers aus Herstellungsbeispiel 1 wurden mit 2,5 g Natriumperchlorat für 1 Stunde auf einer Rollbank homogen vermischt.

Beispiel 4 (erfindungsgemäß):

50 g des wasserabsorbierenden Polymers aus Herstellungsbeispiel 1 wurden mit 2,5 g Natriumperchlorat und 0,125 g HQME für 1 Stunde auf einer Rollbank homogen vermischt.

Von den vorstehenden Zusammensetzungen wurden die ΔL-Werte bestimmt. Die Ergebnisse sind in der nachfolgenden Tabelle zusammengefasst:

| Zusammensetzung | ΔL-Wert |
|---|---|
| Vergleichsbeispiel 2 | 22 |
| Beispiel 4 | 10 |

Die Ergebnisse zeigen, dass HQME auch die durch Natriumperchlorat hervorgerufene starke Verfärbung (siehe Vergleichsbeispiel 2) deutlich zu reduzieren vermag.

Vergleichsbeispiel 3 (Herstellungsbeispiel)

Jeweils 50 g desPulvers A und 50 g des Pulvers B aus dem Herstellungsbeispiels wurden für 20 Tage im bei definierten Bedingungen (70°C, 75% Luffeuchtigkeit) gelagert..

Vergleichsbeispiel 4 (nur Oxidationsmittel, kein Inhibitor):

Jeweils 50 g des wasserabsorbierenden Polymers (Pulver A sowie Pulver B) aus Herstellungsbeispiel 1 wurden mit 1,5 g Natriumpercarbonat (Q35 Standard HPC der Evonik Industries) für 1 Stunde auf einer Rollbank homogen vermischt.

Vergleichsbeispiel 5 (kein Oxidationsmittel, nur Inhibitor):

Jeweils 50 g des wasserabsorbierenden Polymers (Pulver A sowie Pulver B) aus Herstellungsbeispiel 1 wurden mit 0.125 g HQME für 1 Stunde auf einer Rollbank homogen vermischt.

Beispiel 5 (erfindungsgemäß):

Jeweils 50 g des wasserabsorbierenden Polymers (Pulver A sowie Pulver B) aus Herstellungsbeispiel 1 wurden mit 1,5 g Natriumpercarbonat (Q35 Standard HPC der Evonik Industries) und 0,125 g HQME für 1 Stunde auf einer Rollbank homogen vermischt.

Tabelle: ΔL Werte der verschieden behandelten Proben nach Lagerung im Klimaschrank für 20 Tage

| Probe | ΔL-Werte Pulver A | ΔL-Werte Pulver B |
|---|---|---|
| Herstellungsbeispiel 1 | 36 | 9 |
| Vergleichsbeispiel 4 | 52 | 39 |
| Vergleichsbeispiel 5 | 15 | 16 |
| Beispiel 5 | 4 | 12 |

**Patentansprüche**

1. Eine wasserabsorbierende Zusammensetzung, mindestens beinhaltend

> i) 91,2 bis 98,9 Gew.-% mindestens eines wasserabsorbierenden Polymers;
> ii) 1 bis 8 Gew.-% mindestens eines Oxidationsmittels, wobei das Oxidationsmittel ein Percarbonat ist;
> iii) 0,1 bis 0,75 Gew.-% mindestens eines Inhibitors, der radikalische Polymerisationen hemmt, wobei der Inhibitor Hydrochinon oder ein Hydrochinonderivat, ausgewählt aus der Gruppe bestehend aus Hydrochinon, Hydrochinonmonomethylether (HQME), 1,4-Dimethoxybenzol, 4.4'-Oxidiphenol und einer Mischung aus mindestens zwei davon, ist;

> wobei die Gewichtsmengen jeweils auf das Gesamtgewicht der wasserabsorbierenden Zusammensetzung bezogen sind.

2. Wasserabsorbierende Zusammensetzung nach Anspruch 1, wobei das wasserabsorbierende Polymer i) partikulär ist und mindestens 90 Gew.-% der Partikel des wasserabsorbierenden Polymers i) eine Partikelgröße in einem Bereich von 150 bis 850 μm aufweisen.

3. Wasserabsorbierende Zusammensetzung nach einem der vorhergehenden Ansprüche, wobei die Zusammensetzung neben den Komponenten i) bis iii) noch einen Bleichaktivator iv), insbesondere ausgewählt aus der Gruppe bestehend aus Tetraacetylendimain (TAED), Nonanoylbenzolsulphonat (NOBS) und eine Mischung aus mindestens zwei davon, beinhaltet.

4. Wasserabsorbierende Zusammensetzung nach einem der vorhergehenden Ansprüche, wobei die Zusammensetzung einen ΔL-Wert von höchstens 10,0 nach einer Lagerung der wasserabsorbierenden Zusammensetzung für 10 Tage bei 70°C und einer relativen Luftfeuchtigkeit von 75 % aufweist.

5. Ein Verfahren zur Herstellung einer wasserabsorbierenden Zusammensetzung, mindestens beinhaltend die Verfahrensschritte:

> (I) Bereitstellung mindestens eines wasserabsorbierenden Polymers;
> (II) in Kontakt bringen des mindestens einen wasserabsorbierenden Polymers mit mindestens einem Oxidationsmittel, wobei das Oxidationsmittel ein Percarbonat ist;
> (III) in Kontakt bringen des mindestens einen wasserabsorbierenden Polymers mit mindestens einem Inhibitor, der radikalische Polymerisationen hemmt wobei der Inhibitor Hydrochinon oder ein Hydrochinonderivat, ausgewählt aus der Gruppe bestehend aus Hydrochinon, Hydrochinonmonomethylether (HQME), 1,4-Dimethoxybenzol, 4.4'-Oxidiphenol und einer Mischung aus mindestens zwei davon, ist;

wobei Verfahrensschritt III) vor, während oder nach Verfahrensschritt II) durchgeführt werden kann, wobei die relativen Mengen, in denen das wasserabsorbierende Polymer, das Oxidationsmittel und der Inhibitor eingesetzt werden, so bemessen werden, dass eine wasserabsorbierende Zusammensetzung beinhaltend

> i) 91,2 bis 98,9 Gew.-% und am meisten bevorzugt 94,5 bis 97,8 Gew.-% des mindestens einen wasserabsorbierenden Polymers;
> ii) 1 bis 8 Gew.-% und am meisten bevorzugt 2 bis 5 Gew.-% des mindestens einen Oxidationsmittels;
> iii) 0,1 bis 0,75 Gew.-% und am meisten bevorzugt 0,2 bis 0,5 Gew.-% des mindestens einen Inhibitor, der radikalische Polymerisationen hemmt,

erhalten wird, wobei die Gewichtsmengen jeweils auf das Gesamtgewicht der wasserabsorbierenden Zusammensetzung bezogen sind.

6. Verfahren nach Anspruch 5, wobei das wasserabsorbierende Polymer partikulär ist und mindestens 90 Gew.-% der Partikel des wasserabsorbierenden Polymers eine Partikelgröße in einem Bereich von 150 bis 850 μm aufweisen.

7. Verfahren nach einem der Ansprüche 5 bis 6, wobei das Verfahren als weiteren Verfahrensschritt beinhaltet:

> IV) in Kontakt bringen des mindestens einen wasserabsorbierenden Polymers mit mindestens einem Bleichaktivator, insbesondere mit mindestens einem Bleichaktivator ausgewählt aus der Gruppe bestehend aus Tetraacetylendimain (TAED), Nonanoylbenzolsulphonat (NOBS) und eine Mischung aus mindestens zwei davon.

8. Verwendung der wasserabsorbierenden Zusammensetzung nach einem der Ansprüche 1 bis 4 in Schäumen, Formkörpern, Fasern, Folien, Filmen, Kabeln, Dichtungsmaterialien, flüssigkeitsaufnehmenden Hygieneartikeln, Trägern für pflanzen- und pilzwachstumregulierenden Mitteln, Verpackungsmaterialien, Bodenzusätzen, zur kontrollierten Freisetzung von Wirkstoffen oder in Baustoffen.

**Claims**

1. A water-absorbing composition comprising at least

    i) 91.2 to 98.9 wt% of at least one water-absorbing polymer;
    ii) 1 to 8 wt% of at least one oxidizing agent, wherein the oxidizing agent is a percarbonate;
    iii) 0.1 to 0.75 wt% of at least one inhibitor to inhibit free-radical polymerizations, wherein the inhibitor is hydroquinone or a hydroquinone derivative, selected from the group consisting of hydroquinone, hydroquinone monomethyl ether (HQME), 1,4-dimethoxybenzene, 4,4'-oxydiphenol and a mixture of two or more thereof;

    wherein the weight quantities are each based on the overall weight of the water-absorbing composition.

2. The water-absorbing composition according to claim 1 wherein the water-absorbing polymer i) is particulate and at least 90 wt% of the particles of the water-absorbing polymer i) have a particle size in a range from 150 to 850 $\mu$m.

3. The water-absorbing composition according to either preceding claim wherein the composition in addition to the components i) to iii) further comprises a bleach activator iv), in particular selected from the group consisting of tetraacetylenediamine (TAED), nonanoylbenzenesulphonate (NOBS) and a mixture of two or more thereof.

4. The water-absorbing composition according to any preceding claim wherein the composition has a $\Delta L$ value of at most 10.0 after the water-absorbing composition has been stored at 70°C and a relative humidity of 75% for 10 days.

5. A process for producing a water-absorbing composition, comprising at least the steps of:

(I) providing at least one water-absorbing polymer;
(II) contacting the at least one water-absorbing polymer with at least one oxidizing agent, wherein the oxidizing agent is a percarbonate;
(III) contacting the at least one water-absorbing polymer with at least one inhibitor to inhibit free-radical polymerizations, wherein the inhibitor is hydroquinone or a hydroquinone derivative, selected from the group consisting of hydroquinone, hydroquinone monomethyl ether (HQME), 1,4-dimethoxybenzene, 4,4'-oxydiphenol and a mixture of two or more thereof; wherein step III) can be carried out before, during or after step II),

wherein the relative amounts in which the water-absorbing polymer, the oxidizing agent and the inhibitor are used are such as to obtain a water-absorbing composition comprising

    i) 91.2 to 98.9 wt% and most preferably 94.5 to 97.8 wt% of the at least one water-absorbing polymer;
    ii) 1 to 8 wt% and most preferably 2 to 5 wt% of the at least one oxidizing agent;
    iii) 0.1 to 0.75 wt% and most preferably 0.2 to 0.5 wt% of the at least one inhibitor to inhibit free-radical polymerizations;

wherein the weight quantities are each based on the overall weight of the water-absorbing composition.

6. The process according to claim 5 wherein the water-absorbing polymer is particulate and at least 90 wt% of the particles of the water-absorbing polymer have a particle size in a range from 150 to 850 $\mu$m.

7. The process according to either of claims 5 and 6 wherein the process comprises the further step of:

    IV) contacting the at least one water-absorbing polymer with at least one bleach activator, especially with at least one bleach activator selected from the group consisting of tetraacetylenediamine (TAED), nonanoylbenzenesulphonate (NOBS) and a mixture of two or more thereof.

8. The use of the water-absorbing composition according to any of claims 1 to 4 in foams, mouldings, fibres, foils, films, cables, sealing materials, liquid-imbibing hygiene articles, carriers for plant and fungal growth regulators, packaging materials, soil additives, for the controlled release of active ingredients or in building products.

**Revendications**

1. Composition absorbant l'eau, comportant au moins

   i) 91,2 à 98, 9% en poids d'au moins un polymère absorbant l'eau ;
   ii) 1 à 8% en poids d'au moins un oxydant, l'oxydant étant un percarbonate ;
   iii) 0,1 à 0,75% en poids d'au moins un inhibiteur qui inhibe les polymérisations radicalaires, l'inhibiteur étant l'hydroquinone ou un dérivé d'hydroquinone, choisi dans le groupe constitué par l'hydroquinone, l'hydroquinonemonométhyléther (HQME), le 1,4-diméthoxybenzène, le 4,4'-oxydiphénol et un mélange d'au moins deux de ceux-ci ;

   les quantités pondérales se rapportant à chaque fois au poids total de la composition absorbant l'eau.

2. Composition absorbant l'eau selon la revendication 1, le polymère absorbant l'eau i) étant particulaire et au moins 90% en poids des particules du polymère absorbant l'eau i) présentant une grosseur de particule dans une plage de 150 à 850 $\mu$m.

3. Composition absorbant l'eau selon l'une quelconque des revendications précédentes, la composition comportant, outre les composants i) à iii), encore un activateur de blanchiment iv), en particulier choisi dans le groupe constitué par la tétraacétylènediamine (TAED), le nonanoylbenzènesulfonate (NOBS) et un mélange d'au moins deux de ceux-ci.

4. Composition absorbant l'eau selon l'une quelconque des revendications précédentes, la composition présentant une valeur ΔL d'au plus 10,0 après un entreposage de la composition absorbant l'eau pendant 10 jours à 70°C et à une humidité relative de l'air de 75%.

5. Procédé pour la préparation d'une composition absorbant l'eau, comportant au moins les étapes de procédé :

   (I) préparation d'au moins un polymère absorbant l'eau ;
   (II) mise en contact dudit au moins un polymère absorbant l'eau avec au moins un oxydant, l'oxydant étant un percarbonate ;
   (III) mise en contact dudit au moins un polymère absorbant l'eau avec au moins un inhibiteur qui inhibe les polymérisations radicalaires, l'inhibiteur étant l'hydroquinone ou un dérivé d'hydroquinone, choisi dans le groupe constitué par l'hydroquinone, l'hydroquinonemonométhyléther (HQME), le 1,4-diméthoxybenzène, le 4,4'-oxydiphénol et un mélange d'au moins deux de ceux-ci ;

l'étape de procédé III) pouvant être effectuée avant, pendant ou après l'étape de procédé II), les quantités relatives dans lesquelles le polymère absorbant l'eau, l'oxydant et l'inhibiteur sont utilisées étant dimensionnées de manière telle qu'on obtient une composition absorbant l'eau comportant

   i) 91,2 à 98,9% en poids et le plus préférablement 94,5 à 97,8% en poids dudit au moins un polymère absorbant l'eau ;
   ii) 1 à 8% en poids et le plus préférablement 2 à 5% en poids dudit au moins un oxydant ;
   iii) 0,1 à 0,75% en poids et le plus préférablement 0,2 à 0,5% en poids dudit au moins un inhibiteur qui inhibe les polymérisations radicalaires,

les quantités pondérales se rapportant à chaque fois au poids total de la composition absorbant l'eau.

6. Procédé selon la revendication 5, le polymère absorbant l'eau étant particulaire et au moins 90% en poids des particules du polymère absorbant l'eau présentant une grosseur de particule dans une plage de 150 à 850 $\mu$m.

7. Procédé selon l'une quelconque des revendications 5 à 6, le procédé comprenant comme autre étape de procédé :

   IV) la mise en contact dudit au moins un polymère absorbant l'eau avec au moins un activateur de blanchiment, en particulier avec au moins un activateur de blanchiment choisi dans le groupe constitué par la tétraacétylènediamine (TAED), le nonanoylbenzènesulfonate (NOBS) et un mélange d'au moins deux de ceux-ci.

8. Utilisation de la composition absorbant l'eau selon l'une quelconque des revendications 1 à 4 dans des mousses, des corps façonnés, des fibres, des feuilles, des films, des câbles, des matériaux d'étanchéité, des articles hygiéniques absorbant les liquides, des supports pour les agents régulant la croissance des plantes et des champignons, des matériaux d'emballage, des amendements de sol, pour la libération contrôlée de substances actives ou dans des matériaux de construction.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2009066255 A
- EP 0942014 A2
- JP 2005225921 A
- WO 2004085496 A
- WO 03051940 A1
- US 2005085604 A1
- GB 2377890 A
- WO 9908726 A1
- DE 19529348 A1
- WO 2004037903 A2
- US 4286082 A
- DE 2706135 A
- US 4076663 A

- DE 3503458 A
- DE 4020780 C1
- DE 4244548 A
- DE 4333056 A
- DE 4418818 A
- DE A2706135 A1
- WO 2004037903 A
- US 4340706 A
- DE 3713601 A
- DE 2840010 A
- WO 9605234 A
- WO 2005080542 A
- WO 02056812 A1

**In der Beschreibung aufgeführte Nicht-Patentliteratur**

- **F. L. BUCHHOLZ ; A. T. GRAHAM.** Modem Super-absorbent Polymer Technology. Wiley-VCH, 1998